# EUROPEAN PATENT SPECIFICATION

(11) **EP 1 736 866 B1**
(45) Date of publication and mention of the grant of the patent: **28.07.2010**
(21) Application number: 06115882.0
(22) Date of filing: 22.06.2006
(51) Int. Cl.: G06F 3/14

(54) **Display apparatus with a network connection**
Anzeigevorrichtung mit Netzwerkanschluss
Appareil d'affichage avec une connection réseau

(30) Priority: 25.06.2005 KR 2005055350
(43) Date of publication of application: 27.12.2006
(73) Proprietor: Samsung Electronics Co., Ltd, Suwon-si, Gyeonggi-do 442-742 (KR)
(72) Inventor: Ahn, Young-ho, Seoul (KR)
(74) Representative: Perkins, Dawn Elizabeth

(56) References cited:
- EP-A- 1 257 125
- EP-A- 1 381 187
- US-A1- 2004 117 844
- US-A1- 2004 160 408
- US-A1- 2006 026 302

## Description

The present invention relates to a display apparatus, and more particularly, to a display apparatus, which transmits and receives data through a network, and reduces power consumption.

Generally, a display apparatus includes a liquid crystal display (LCD), a plasma display panel (PDP), etc. The display apparatus is employed as a monitor of a TV or a computer to display images thereon.

If the display apparatus is used as a monitor, the display apparatus performs various functions such as receiving TV broadcast signals through a tuner and displaying them thereon, outputting sound signals from the computer through an attached speaker, and displaying video signals received from the computer thereon as images. Such a monitor having various functions is referred to as a multi-function monitor.

Meanwhile, monitors and computers are connected to each other through a D-Sub connector or a DVI connector to allow the video signals from the computer to be transmitted to the monitor.

The multi-function monitor may comprise an S-video connector, a component connector or a composite connector, as well as the D-Sub connector or the DVI connector mentioned above, to receive a video signal from external devices such as a digital video disk (DVD) player or a videocassette recorder (VCR).

Also, the computer has more than a single output connector, such as the D-Sub connector, that is, the computer has two or more output connectors, such as the D-Sub connector, the DVI connector, the S-video connector, etc. Thus, the computer may be connected with two or more monitors.

The recent multi-function monitor provides various functions such as displaying data received through the network as images, sending and receiving a predetermined control signal through the network, as well as being connected with the computer through a video cable.

Meanwhile, various power saving modes are provided to reduce power consumption of the multi-function monitor. For example, according to a display power management signalling (DPMS) of video electronics standard association (VESA), the multi-function monitor is converted into a power saving mode depending on conditions of the video signals received from the computer (e.g., according to whether a synchronous signal is supplied).

However, the multi-function monitor having the network function does not have an additional power saving mode when processing data received through the network.

It becomes necessary to provide a monitor having the network function to operate in a power saving mode according to operating conditions of the monitor and to reduce power consumption thereof.

US 2004/0117844 discloses a display communicatively coupled to a communication device and a network. The communication device may be in a "standby" mode and an "off" mode.

EP 1 381 187 discloses an electronic apparatus having an audio-video (AV) function section and an embedded controller for shifting or recovering the AV function section from standby mode. A network processor accepts, via a network such as a wireless LAN, a command, on receipt of which the network instructs, via a control line, the embedded controller to shift or recover the AV function to or from the standby mode.

The present invention is defined in the claims.

These and/or other aspects and advantages of the present invention will become apparent and more readily appreciated from the following description of the embodiments, taken in conjunction with the accompanying drawings of which:
Figure 1 illustrates a connection between a display apparatus according to an embodiment of the present invention and a computer; and
Figures 2 and 3 are control block diagrams of the display apparatus of Figure 1.

Reference will now be made in detail to the embodiments of the present invention, examples of which are illustrated in the accompanying drawings, wherein like reference numeral refer to the like elements throughout. The embodiments are described below in order to explain the present invention by referring to the figures.

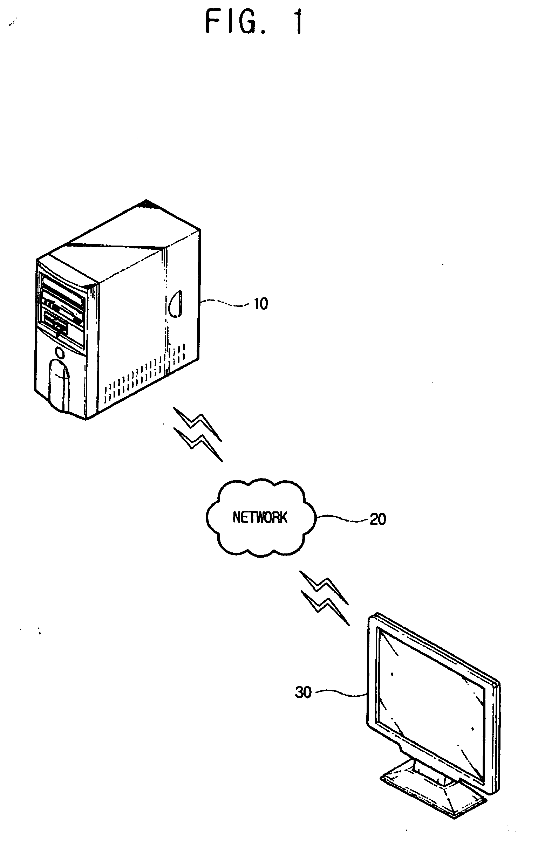

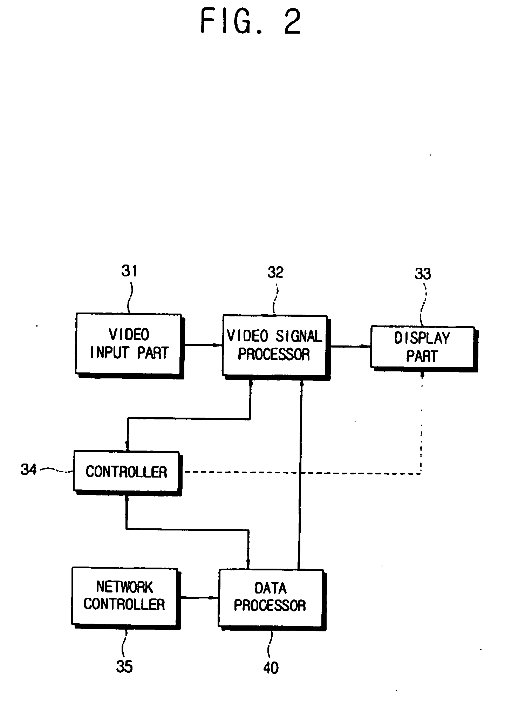

As illustrated in Figure 1, a display apparatus 30 according to an embodiment of the present invention is connected with an external device, e.g., with a computer 10 through a network 20. The display apparatus 30 according to the present embodiment may further comprise a video input part 31 to connect with the computer 10 through a video cable to transmit a video signal from the computer 10 to the display apparatus 30 according to a predetermined video format, as illustrated in Figure 2. Here, the video input part 31 may comprise at least one of a D-Sub connector, a DVI connector, a S-video connector, a component connector and a composite connector.

Referring to Figure 2, the display apparatus 30 according to the present embodiment further comprises a display part 33, a video signal processor 32, a network controller 35, a data processor 40 and a controller 34.

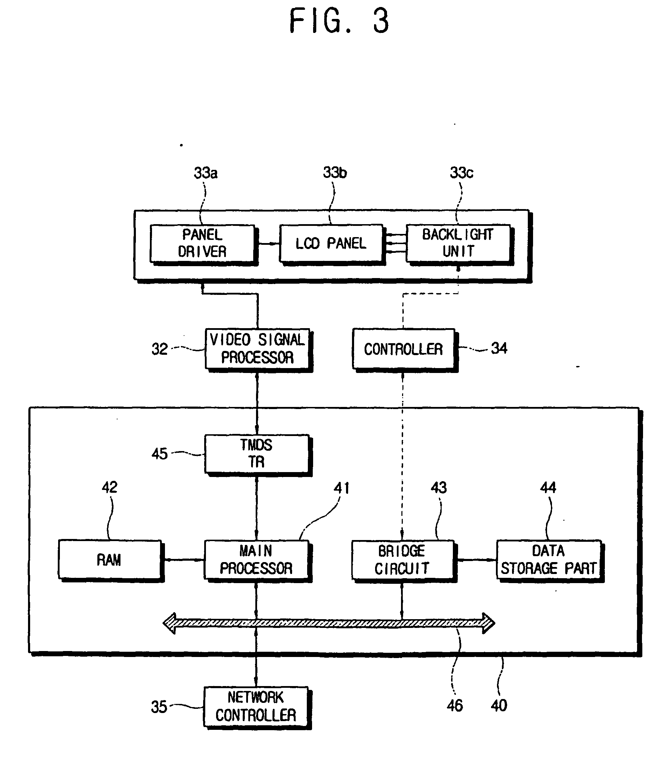

The display part 33 displays the video signal received from the video signal processor 32 as an image thereon. As illustrated in Figure 3, the display part 33 according to the present embodiment employs a liquid crystal display (LCD) comprising a liquid crystal display (LCD) panel 33b and a backlight unit 33c to emit light toward the LCD panel 33b, as an illustration of the present embodiment. However, it should be understood that the above-described embodiment of the display part is merely illustrative and is not intended to be limiting to the present invention. According to the embodiment illustrated in Figure 3, the display part 33 comprises a panel driver 33a to drive the LCD panel 33b according to the video signal from the video signal processor 32.

The video signal processor 32 converts the video signal input from the video input part 31 or the data processor 40 into a format displayable by the display part 33 before supplying the video signal to the display part 33. The video signal supplied from the video input part 31 to the video signal processor 32 is referred to as a first video signal. The video signal supplied from the data processor 40 to the video signal processor 32 is referred to as a second video signal.

The video signal processor 32 may be provided in various forms corresponding to various formats of the input video signal. For example, the video signal processor 32 may comprise a scaler, and a video signal converter to convert the first video signal received in various formats, which is input through the video input part 31 in a format processible by the scaler.

Here, the video signal converter 32 may comprise at least one of an A/D converter, a video decoder and a tuner, corresponding to the various formats in which the first video signal may be received. The scaler converts the video signal output from the video signal converter to conform to a vertical frequency, resolution, an image ratio of output standards of the display part 33.

The network controller 35 is connected to the network 20 to send and receive data according to a predetermined protocol. The network controller 35 according to the present embodiment may be connected with the computer 10 through a local area network (LAN) wirelessly or through a network cable. Also, the network controller 35 may be connected with the computer 10 using other methods, such as infrared communication or bluetooth, as long as the network controller communicates with the computer 10.

The data processor 40 processes the data transmitted and received through the network controller 35. The data processor 40 outputs a power saving mode conversion signal according to predetermined conditions.

The controller 34 controls the video signal processor 32 and the display part 33 to display the image on the display part 33. Also, the controller 34 controls the display apparatus 30 to convert the display apparatus 30 into a preset power saving mode according to the received power saving mode conversion signal if the power saving mode conversion signal is received from the data processor 40.

Hereinafter, the display apparatus 30 according to an embodiment of the present invention will be described in detail with reference to Figure 3.

The data processor 40 according to the present embodiment may comprise a data storage part 44, a RAM 42 as a memory, a main processor 41 and a bridge circuit 43.

The data storage part 44 stores at least one control routine therein to process data received through the network controller 35. For example, the at least one control routine stored in the data storage part 44 may comprise an operating system to drive the data processor 40, and an application program to process various data received through the network controller 35.

Here, the application program may comprise a codec program to decompress graphics data received from the network controller 35 and compressed into a predetermined format, and a processing program to process the graphics data decompressed through the codec program.

The main processor 41 loads the control routine stored in the data storage part 44 to the memory 42 to execute the control routine. The main processor 41 may be provided as a one chip processor comprising a central processing unit (CPU), a memory controller to control the memory 42, and a graphics controller to process the video signal.

The video signal output by the main processor 41 is converted into the transition minimized differential signalling (TMDS) type second video signal through a transition minimized differential signalling (TMDS) transmitter 45 to be output to the video signal processor 32.

The bridge circuit 43 allows the data storage part 44 and the main processor 41 to exchange data with each other. The main processor 41 and the bridge circuit 43 may be connected with each other through a predetermined bus, for example, a peripheral component interconnect (PCI) bus 46.

Meanwhile, the controller 34 is connected with the bridge circuit 43 to receive the power saving mode conversion signal from the bridge circuit 43.

The power saving mode conversion signal is supplied to the controller 34 through the bridge circuit 43 if the main processor 41 satisfies predetermined conditions. For example, the main processor 41 may output the power saving mode conversion signal corresponding to the preset power saving mode through the bridge circuit 43 if the preset power saving mode supported by the operating system stored in the data storage part 44 is executed.

At this time, the controller 34 converts the display apparatus 30 into the preset power saving mode according to the received power saving mode conversion signal if the power saving mode conversion signal is received from the bridge circuit 43. For example, the controller 34 turns off the backlight unit 33c according to the received power saving mode conversion signal if the power saving mode conversion signal is received. Accordingly, the display apparatus 30 according to the present embodiment may reduce power consumption.

The main processor 41 supplies a normal mode conversion signal to the controller 34 through the bridge circuit 43 if, for example, the data processor 40 receives new input, after the controller 34 has converted the display apparatus 30 into the preset power saving mode according to the power saving mode conversion signal. The controller 34 converts the display apparatus 30 into a normal mode according to the normal mode conversion signal, e.g., turns on the backlight unit 33c.

Thus, the display apparatus 30 may be selectively converted into the preset power saving mode and the normal mode according to an operating state of the data processor 40.

Meanwhile, the data processor 40 according to the present embodiment may be driven by an operating system and an application program stored in the computer 10 (i.e. an external device connected through the network 20), instead of being driven by the operating system and the application program stored in the data storage part 44.

That is, the main processor 41 of the data processor 40 may execute the operating system and the application program of the computer 10, and may receive desired data, such as the graphics data, through the network 20. The data processor 40 may control the operating system or the application program by receiving a command through a keyboard or a mouse connected through a USB and by transmitting the command to the computer 10 through the network 20.

If the operating system of the computer 10 is converted into a predetermined power saving mode, (e.g., if a screen saver is activated) the main processor 41 may receive a first control signal corresponding to the predetermined power saving mode from the computer 10 through the network controller 35 and may supply the power saving mode conversion signal to the controller 34 corresponding to a reception of the first control signal.

If the computer 10 is converted from the predetermined power saving mode to an active mode (e.g., the screen saver is inactivated), the main processor 41 receives a second control signal corresponding to the active mode from the computer 10 through the network controller 35, and supplies the normal mode conversion signal to the controller 34 through the bridge circuit 43.

Accordingly, even if the data processor 40 operates based on the operating system or the application program of the computer 10, the display apparatus 30 may operate in the preset power saving mode and the normal mode.

Meanwhile, the controller 34 according to the present embodiment may operate in a first display mode to control the video signal processor 32 to display the first video signal input through the video input part 31 as the image on the display part 33, and in a second display mode to control the video signal processor 32 to display the second video signal output from the data processor 40 as the image on the display part 33.

Here, the controller 34 remains in the normal mode if the power saving mode conversion signal is received from the data processor 40 when the controller 34 operates in the first display mode. That is, the controller 34 allows the first video signal input through the video input part 31 to continue to be displayed as the image on the display part 33.

Conversely, the controller 34 converts the display apparatus 30 into the preset power saving mode if the power saving mode conversion signal is received from the data processor 40 when the controller 34 operates in the second display mode.

Also, the controller 34 may operate in the preset power saving mode according to a state of the first video signal input through the video input part 31 if the controller 34 operates in the first display mode. The preset power saving mode operated according to the state of the first video signal may comprise a display power management signaling (DPMS) of video electronics standard association (VESA).

Thus, the controller 34 of the display apparatus 30 according to embodiments of the present invention operates individually in the first display mode and the second display mode, thereby efficiently reducing power consumption according to the operating state of respective modes.

The embodiments of the present invention can also be embodied as computer readable codes on a computer readable recording medium. The computer readable recording medium may include any data storage device that can store data which can be thereafter read by a computer system. Examples of the computer readable recording medium include a read-only memory (ROM), a random-access memory (RAM), CD-ROMs, magnetic tapes, floppy disks, optical data storage devices, and carrier waves (such as data transmission through the Internet). The computer readable recording medium can also be distributed over network coupled computer systems so that the computer readable code is stored and executed in a distributed fashion. The embodiments of the present invention may also be embodied in hardware or a combination of hardware and software. For example, the data processor 40 and the controller 30 may be embodied in software, hardware, or a combination thereof.

Although a few embodiments of the present invention have been shown and described, it will be appreciated by those skilled in the art that changes may be made in these embodiments without departing from the principles of the invention, the scope of which is defined in the appended claims.

## Claims

1. A display apparatus (30) comprising:
a display part (33);
a video input part (31) arranged to receive a first video signal in a first predetermined format;
a network controller (35) connectable to a network (20) and arranged to transmit and receive data according to a predetermined protocol;
a data processor (40) arranged to process said data transmitted and received through said network controller (35) to extract from said received data a second video signal in a second predetermined format, and to output a power saving mode conversion signal depending on the received data;
a video signal processor (32) arranged to convert said first video signal or said second video signal into a format displayable by said display part (33) and to output a converted first video signal or a converted second video signal to said display part (33); said display apparatus (30) being **characterized in that** it comprises :
a controller (34) arranged in a first, display mode, to control said video signal processor (32) to output said converted first video signal and, in a second display mode, to control said video signal processor (32) to output said converted second video signal, wherein, in said first display mode, said controller (34) controls said display apparatus (30) to remain in a normal mode if said power saying mode conversion signal is received from said data processor (40) and, in said second display mode, said controller (34) controls said display apparatus (36) to operate in a power saving mode if said power saving mode conversion signal is received from said data processor (40).

2. The display apparatus according to claim 1, wherein the display part (33) comprises:
a liquid crystal display (LCD) panel (33b) ; and
a backlight unit (33c) configured to emit light toward the LCD panel (33b), wherein the controller (34) is configured to turn off the backlight unit (33c) according to the received power saving mode conversion signal.

3. The display apparatus according to claim 1 or 2, wherein the controller (34) is configured to control the display apparatus (30) to operate in a normal mode if a normal mode conversion signal is received from the data processor (40) when the display apparatus (30) is in the power saving mode.

4. The display apparatus according to any one of the preceding claims, wherein the data processor (40) comprises:
a data storage part (44) having at least one control routine stored therein to process the data received through the network controller (35);
a memory (42) configured to load the at least one control routine and an operating system;
a a main processor (41) configured to load the at least one control routine to the memory (42) and to execute the at least one control routine; and
a bridge circuit (43) configured to allow the data storage part (44) and the main processor (41) to communicate with each other.

5. The display apparatus according to claim 4, wherein the controller (34) is connected to the bridge circuit (43), and the main processor (41) is configured to selectively supply the power saving mode conversion signal and the normal mode conversion signal to the controller (34) through the bridge circuit (43).

6. The display apparatus according to claim 4 or 5, wherein the main processor (41) is configured to supply one of the power saving mode conversion signal and the normal mode conversion signal to the controller (34) based on a control signal received through the network controller (35) from an external device (10) connected through the network (20).

7. The display apparatus according to any one of the preceding claims, wherein the controller (34) converts the display apparatus (30) to operate in the power saving mode according to a state of the first video signal input through the video input part (31) while operating in the first display mode.

8. The display apparatus according to claim 7, wherein the power saving mode according to the state of the first video signal comprises a display power management signalling (DPMS) of video electronics standard association (VESA).

9. A method of controlling a display apparatus (30) comprising the steps of:
receiving a first video signal in a first predetermined format;
receiving data from a network (20) via a network controller (35) according to a predetermined protocol;
processing said data received from the network (20) and extracting from said received data a second video signal in a second predetermined format;
providing a power saving mode conversion signal to a controller (34) depending on said received data;
converting said first video signal or said second video signal in a video signal processor (32) into a format displayable by a display part (33) and outputting a converted first video signal or a converted second video signal to said display part (33); said method being **characterized in that** :
said controller (34) is arranged to said video signal processor (32) to output said converted first video signal in a first display mode or said converted second video signal in a second display mode, and, in said first display mode, to controling said display apparatus (30) to remain in a normal mode if said power saving mode conversion signal is received by said controller (34), and, in said second display mode, to control said display apparatus (30) to operate in a power saving mode if said power saving mode conversion signal is received by said controller (34).

10. A computer readable medium containing executable code which, when executed on a computer, controls a display apparatus (30) connected to a network (20) to carry out the method according to claim 9.

## Patentansprüche

1. Anzeigeapparat (30) umfassend:
ein Anzeigeteil (33);
ein Videoeingabeteil (31), das zum Empfangen eines ersten Videosignals in einem ersten vorbestimmten Format angeordnet ist;
einen Netzwerkregler (35), der an ein Netzwerk (20) anschließbar und so angeordnet ist, dass er Daten einem vorbestimmten Protokoll entsprechend überträgt und empfängt;
einen Datenprozessor (40), der so angeordnet ist, dass er die Daten verarbeitet, die durch den Netzwerkregler (35) übertragen und empfangen werden, aus den empfangenen Daten ein zweites Videosignal in einem zweiten vorbestimmten Format extrahiert und ein Stromsparmodus-Umwandlungssignal je nach den empfangenen Daten ausgibt;
einen Videosignalprozessor (32), der so angeordnet ist, dass er das erste Videosignal oder das zweite Videosignal in ein Format umwandelt, das durch das Anzeigeteil (33) anzeigbar ist, und ein umgewandeltes erstes Videosignal oder ein umgewandeltes zweites Videosignal zu dem Anzeigeteil (33) ausgibt; wobei der Anzeigeapparat (30) **dadurch gekennzeichnet ist, dass** er Folgendes umfasst:
einen Regler (34) der in einem ersten Anzeigemodus so angeordnet ist, dass er den Videosignalprozessor (32) regelt, um das umgewandelte erste Videosignal auszugeben und in einem zweiten Anzeigemodus den Videosignalprozessor (32) regelt, um das umgewandelte zweite Videosignal auszugeben, wobei im ersten Anzeigemodus der Regler (34) den Anzeigeapparat (30) so regelt, dass er in einem normalen Modus bleibt, wenn das Stromsparmodus-Umwandlungssignal von dem Datenprozessor (40) empfangen wird und in dem zweiten Anzeigemodus der Regler (34) den Anzeigeapparat (30) so regelt, dass er in einem Stromsparmodus arbeitet, wenn das Stromsparmodus-Umwandlungssignal von dem Datenprozessor (40) empfangen wird.

2. Anzeigeapparat nach Anspruch 1, wobei das Anzeigeteil (33) Folgendes umfasst:
eine Flüssigkristallanzeige- (LCD-) Tafel (33b); und
eine Hinterleuchtungseinheit (33c), die so konfiguriert ist, dass sie Licht auf die LCD-Tafel (33b) zu emittiert, wobei der Regler (34) so konfiguriert ist, dass er die Hinterleuchtungseinheit (33c) dem empfangenen Stromsparmodus-Umwandlungssignal entsprechend abstellt.

3. Anzeigeapparat nach Anspruch 1 oder 2, wobei der Regler (34) so konfiguriert ist, dass er den Anzeigeapparat (30) so regelt, dass er in einem normalen Modus arbeitet, wenn ein Normalmodus-Umwandlungssignal vom Datenprozessor (40) empfangen wird, wenn der Anzeigeapparat (30) sich im Stromsparmodus befindet.

4. Anzeigeapparat nach einem der vorhergehenden Ansprüche, wobei
der Datenprozessor (40) Folgendes umfasst:
ein Datenspeicherteil (44), in dem mindestens eine Regelroutine gespeichert ist, um die durch den Netzwerkregler (35) empfangenen Daten zu verarbeiten;
einen Speicher (42), der so konfiguriert ist, dass er die mindestens eine Regelroutine und ein Betriebssystem ladet;
einen Hauptprozessor (41), der so konfiguriert ist, dass der die mindestens eine Kontrollroutine in den Speicher (42) ladet und die mindestens eine Regelroutine durchführt; und
eine Brückenschaltung (43), die so konfiguriert ist, dass sie es dem Datenspeicherteil (44) und dem Hauptprozessor (41) erlaubt, mit einander zu kommunizieren.

5. Anzeigeapparat nach Anspruch 4, wobei der Regler (34) an die Brückenschaltung (43) angeschlossen ist und der Hauptprozessor (41) so konfiguriert ist, dass er das Stromsparmodus-Umwandlungssignal und das Normalmodus-Umwandlungssignal selektiv an den Regler (34) durch die Brückenschaltung (43) liefert.

6. Anzeigeapparat nach Anspruch 4 oder 5, wobei der Hauptprozessor (41) so konfiguriert ist, dass er eines von dem Stromsparmodus-Umwandlungssignal und dem Normalmodus-Umwandlungssignal an den Regler (34) auf der Basis eines Kontrollsignals liefert, das durch den Netzwerkregler (35) von einem externen Gerät (10), das durch das Netzwerk (20) angeschlossen ist, empfangen wird.

7. Anzeigeapparat nach einem der vorhergehenden Ansprüche, wobei der Regler (34) den Anzeigeapparat (30) umwandelt, um im Stromsparmodus einem Zustand der ersten Videosignaleingabe durch das Videoeingabeteil (31) entsprechend zu arbeiten, während er im ersten Anzeigemodus arbeitet.

8. Anzeigeapparat nach Anspruch 7, wobei der Stromsparmodus dem Zustand des ersten Videosignals entsprechend ein Display Power Management Signalling (DPMS) der Video Electronics Standard Association (VESA) umfasst.

9. Methode zum Regulieren eines Anzeigeapparats (30), umfassend die Schritte des:
Empfangens eines ersten Videosignals in einem ersten vorbestimmten Format;
Empfangens von Daten von einem Netzwerk (20) über einen Netzwerkregler (35) einem vorbestimmten Protokoll entsprechend;
Verarbeitens der von dem Netzwerk (20) empfangenen Daten und Extrahierens aus den empfangenen Daten eines zweiten Videosignals in einem zweiten vorbestimmten Format;
Bereitstellens eines Stromsparmodus-Umwandlungssignals an einen Regler (34) je nach den empfangenen Daten;
Umwandelns des ersten Videosignals oder des zweiten Videosignals in einem Videosignalprozessor (32) in ein Format, das durch ein Anzeigeteil (33) anzeigbar ist und Ausgebens eines umgewandelten ersten Videosignals oder eines umgewandelten zweiten Videosignals an das Anzeigeteil (33); wobei die Methode **dadurch gekennzeichnet ist, dass**:
der Regler (34) so angeordnet ist, dass er den Videosignalprozessor (32) so regelt, dass er das umgewandelte erste Videosignal in einem ersten Anzeigemodus oder das umgewandelte zweite Videosignal in einem zweiten Anzeigemodus ausgibt und in dem ersten Anzeigemodus den Anzeigeapparat (30) so regelt, dass er in einem normalen Modus bleibt, wenn das Stromsparmodus-Umwandlungssignal vom Regler (34) empfangen wird und in dem zweiten Anzeigemodus den Anzeigeapparat (30) so regelt, dass er in einem Stromsparmodus arbeitet, wenn das Stromsparmodus-Umwandlungssignal vom Regler (34) empfangen wird.

10. Computerlesbares Medium, das eine ausführbare Code enthält, die, wird sie auf einem Computer ausgeführt, einen Anzeigeapparat (30) regelt, der an ein Netzwerk (20) angeschlossen ist, um die Methode nach Anspruch 9 durchzuführen.

## Revendications

1. Appareil de visualisation (30), comprenant :
une partie de visualisation (33) ;
une partie d'entrée vidéo (31) agencée pour recevoir un premier signal vidéo dans un premier format prédéfini ;
un contrôleur de réseau (35) connectable à un réseau (20) et agencé pour transmettre et recevoir des données selon un protocole prédéfini ;
un processeur de données (40) agencé pour traiter lesdites données transmises et reçues par le biais dudit contrôleur de réseau (35), pour extraire desdites données reçues un deuxième signal vidéo dans un deuxième format prédéfini, et pour délivrer un signal de passage en mode d'économie d'énergie en fonction des données reçues ;
un processeur de signal vidéo (32) agencé pour convertir ledit premier signal vidéo ou ledit deuxième signal vidéo en un format affichable par ladite partie de visualisation (33) et pour délivrer un premier signal vidéo converti ou un deuxième signal vidéo converti à ladite partie de visualisation (33) ;
ledit appareil de visualisation (30) étant **caractérisé en ce qu'**il comprend :
un contrôleur (34) agencé, dans un premier mode de visualisation, pour commander ledit processeur de signal vidéo (32) afin qu'il délivre ledit premier signal vidéo converti, et dans un deuxième mode de visualisation, pour commander ledit processeur de signal vidéo (32) afin qu'il délivre ledit deuxième signal vidéo converti, dans lequel, dans ledit premier mode de visualisation, ledit contrôleur (34) commande ledit appareil de visualisation (30) de façon qu'il reste dans un mode normal si ledit signal de passage en mode d'économie d'énergie est reçu dudit processeur de données (40) et, dans ledit deuxième mode de visualisation, ledit contrôleur (34) commande ledit appareil de visualisation (30) de façon qu'il fonctionne dans un mode d'économie d'énergie si ledit signal de passage en mode d'économie d'énergie est reçu dudit processeur de données (40).

2. Appareil de visualisation selon la revendication 1, dans lequel la partie de visualisation (33) comprend :
un écran de visualisation à cristaux liquides (LCD) (33b) ; et
un module de rétro-éclairage (33c) configuré pour émettre de la lumière en direction de l'écran LCD (33b), dans lequel le contrôleur (34) est configuré pour désactiver le module de rétro-éclairage (33c) suivant le signal de passage en mode d'économie d'énergie reçu.

3. Appareil de visualisation selon la revendication 1 ou la revendication 2, dans lequel le contrôleur (34) est configuré pour commander l'appareil de visualisation (30) de façon qu'il fonctionne dans un mode normal si un signal de passage en mode normal est reçu du processeur de données (40) lorsque l'appareil de visualisation (30) est en mode d'économie d'énergie.

4. Appareil de visualisation selon l'une quelconque des revendications précédentes, dans lequel le processeur de données (40) comprend :
une partie de stockage de données (44) dans laquelle au moins une routine de commande est mémorisée afin de traiter les données reçues par le biais du contrôleur de réseau (35) ;
une mémoire (42) configurée pour charger ladite au moins une routine de commande ainsi qu'un système d'exploitation ;
un processeur principal (41) configuré pour charger ladite au moins une routine de commande dans la mémoire (42) et pour exécuter ladite au moins une routine de commande ; et
un circuit de passerelle (43) configuré pour permettre à la partie de stockage de données (44) et au processeur principal (41) de communiquer entre eux.

5. Appareil de visualisation selon la revendication 4, dans lequel le contrôleur (34) est relié au circuit de passerelle (43) et le processeur principal (41) est configuré pour délivrer sélectivement le signal de passage en mode d'économie d'énergie et le signal de passage en mode normal au contrôleur (34) par le biais du circuit de passerelle (43).

6. Appareil de visualisation selon la revendication 4 ou la revendication 5, dans lequel le processeur principal (41) est configuré pour délivrer au contrôleur (34) un signal choisi parmi le signal de passage en mode d'économie d'énergie et le signal de passage en mode normal sur la base d'un signal de commande reçu par le biais du contrôleur de réseau (35) à partir d'un dispositif externe (10) connecté par le biais du réseau (20).

7. Appareil de visualisation selon l'une quelconque des revendications précédentes, dans lequel le contrôleur (34) convertit l'appareil de visualisation (30) de façon qu'il fonctionne en mode d'économie d'énergie en fonction d'un état du premier signal d'entrée vidéo reçu par le biais de la partie d'entrée vidéo (31) lorsqu'il fonctionne dans le premier mode de visualisation.

8. Appareil de visualisation selon la revendication 7, dans lequel le mode d'économie d'énergie en fonction de l'état du premier signal vidéo comprend une signalisation DPMS (*display power management signalling*) selon le standard VESA *(Video Electronics Standard Association).*

9. Procédé de commande d'un appareil de visualisation (30), comprenant les étapes consistant à :
recevoir un premier signal vidéo dans un premier format prédéfini ;
recevoir des données provenant d'un réseau (20) par le biais d'un contrôleur de réseau (35) selon un protocole prédéfini ;
traiter lesdites données reçues à partir du réseau (20) et extraire desdites données reçues un deuxième signal vidéo dans un deuxième format prédéfini ;
délivrer un signal de passage en mode d'économie d'énergie à un contrôleur (34) en fonction desdites données reçues ;
convertir ledit premier signal vidéo ou ledit deuxième signal vidéo dans un processeur de signal vidéo (32) en un format affichable par une partie de visualisation (33) et délivrer un premier signal vidéo converti ou un deuxième signal vidéo converti à ladite partie de visualisation (33) ;
ledit procédé étant **caractérisé en ce que** :
ledit contrôleur (34) est agencé pour commander ledit processeur de signal vidéo (32) de façon qu'il délivre ledit premier signal vidéo converti dans un premier mode de visualisation ou ledit deuxième signal vidéo converti dans un deuxième mode de visualisation, et, dans ledit premier mode de visualisation, pour commander ledit appareil de visualisation (30) de façon qu'il reste dans un mode normal si ledit signal de passage en mode d'économie d'énergie est reçu par ledit contrôleur (34) et, dans ledit deuxième mode de visualisation, pour commander ledit appareil de visualisation (30) de façon qu'il fonctionne dans un mode d'économie d'énergie si ledit signal de passage en mode d'économie d'énergie est reçu par ledit contrôleur (34).

10. Support lisible informatiquement, contenant un code exécutable qui, lorsqu'il est exécuté sur un ordinateur, commande l'appareil de visualisation (30) relié à un réseau (20) de façon à réaliser le procédé de la revendication 9.
